# EUROPEAN PATENT APPLICATION

(11) **EP 3 456 380 A1**
(43) Date of publication of application: **20.03.2019**
(21) Application number: 17191406.2
(22) Date of filing: 15.09.2017
(51) Int. Cl.: A61N 5/06

(54) **MATERIAL CAPABLE OF EMITTING RADIATION IN THE UV-B WAVELENGTH RANGE**

(71) Applicant: Seaborough Life Science B.V., 1019 AG Amsterdam (NL)
(72) Inventor: Meijerink, Andries, 3769 DG Soesterberg (NL); van de Haar, Marie Anne, 1381 HL Weesp (NL); Hilgerink, Tom, 1093 NH Amsterdam (NL); Honold, Jürgen Eduard, 1087 JC Amsterdam (NL)
(74) Representative: Hoyng Rokh Monegier LLP

(57) **Abstract**

The invention relates to a material capable of emitting radiation in the UV-B wavelength range (between 290 and 320 nm), preferably in the wavelength range between 305 nm and 315 nm, upon excitation by radiation in the wavelength range between 400 nm and 495 nm. The invention also relates to a system, a kit of parts and method for generating UV-B radiation involving the use of this material. The invention further relates to a method of exposing cells and/or cell tissue to UV-B radiation, in particular for therapeutic treatment such as the treatment of psoriasis.

## Description

### FIELD OF THE INVENTION

The invention relates to a material capable of emitting radiation in the UV-B wavelength range and to a phosphor capable of emitting radiation in the UV-B wavelength range. The invention also relates to a system, a kit of parts and method for generating UV-B radiation, as well as to the use of the material and phosphor for generating UV-B radiation. The invention further relates to a method of exposing cells and/or cell tissue to UV-B radiation, in particular for therapeutic treatment such as the treatment of skin disease, for instance psoriasis.

### BACKGROUND OF THE INVENTION

Light therapy is a treatment method that has been practiced since multiple centuries in the medical field of dermatology. The Egyptians, Romans and Greeks already noticed that patients suffering from certain types of skin disorders can benefit from sunlight. Nowadays light therapy is most often performed using narrow band UV-B emitting artificial light sources.

Although narrow band UV-B light therapy may offer treatment, there is a strong discrepancy between patients that are eligible for phototherapy and the actual number of users. This discrepancy can mainly be attributed to the (in)availability of phototherapy, long term treatment plans, time consuming sessions and inability of the patients to perform the treatment by themselves.

Apart from the fact that phototherapy is often inconvenient to fit into the daily life schedules of patients, it also has some intrinsic (safety) drawbacks limiting the effectiveness of the treatment. Most side effects are related to UV-B exposure of healthy skin surrounding the affected area(s) due to light spill. This causes unnecessary health risks and at the same time slows down treatment times as erythema effects on the healthy skin are usually the limiting factor in terms of radiation doses that can be applied.

In principle these side effects could be minimized by using UV blockers on the healthy skin. However, this is often a cumbersome solution since many patients have multiple affected skin areas, which requires full-body exposure treatments.

US 2013/031730 describes an approach which involves application of a covering composition such as a photocream which filters certain wavelengths and selectively allows passage of radiation within a predetermined wavelength band, e.g. in the UV-B range. Thus, healthy skin may still be exposed to UV-B radiation.

Alternatively blue light therapy can be used as a safer method with less side effects, which is a popular approach for home treatments. However, blue light is much less effective for the treatment of most skin diseases, requiring much longer exposure times and less relieve for the patients.

In view of these problems, it is an object of the invention to provide a method and system that can be safely applied at home and in a clinical environment and is less harmful for healthy skin.

### SUMMARY OF THE INVENTION

The invention provides a material capable of emitting radiation in the UV-B wavelength range (between 290 and 320 nm), preferably in the wavelength range between 305 nm and 315 nm, upon excitation by radiation in the wavelength range between 400 nm and 495 nm.

The invention further provides a phosphor comprising a host lattice doped with at least (i) first lanthanide ions which are Gd³⁺ ions and (ii) second lanthanide ions selected from the group consisting of Ho³⁺, Tm³⁺, Er³⁺, Dy³⁺ and combinations thereof.

The material or phosphor according to the invention may be excited by radiation in the wavelength range between 400 nm and 495 nm, to thereby generate radiation in the UV-B wavelength range.

The invention enables to combine the safety of radiation in the wavelength range between 400 nm and 495 nm with the high effectiveness of UV-B radiation. The material or phosphor or compositions comprising the material or phosphor according to the invention may be applied locally, thereby enabling to apply UV-B radiation locally in a controlled way. Radiation in the wavelength range between 400 nm and 495 nm allows replacement of legacy UV-B light sources for often more stable more efficient sources, e.g. blue LED. Whilst the phenomenon of upconversion, i.e. the creation of a high energy photon absorption of multiple lower energy photons has been known, for instance the upconversion from NIR to the visible range, the present invention shows that upconversion from the wavelength range between 400 nm and 495 nm to radiation in the UV-B wavelength range (290 nm to 320 nm) is possible.

### BRIEF DESCRIPTION OF THE DRAWINGS

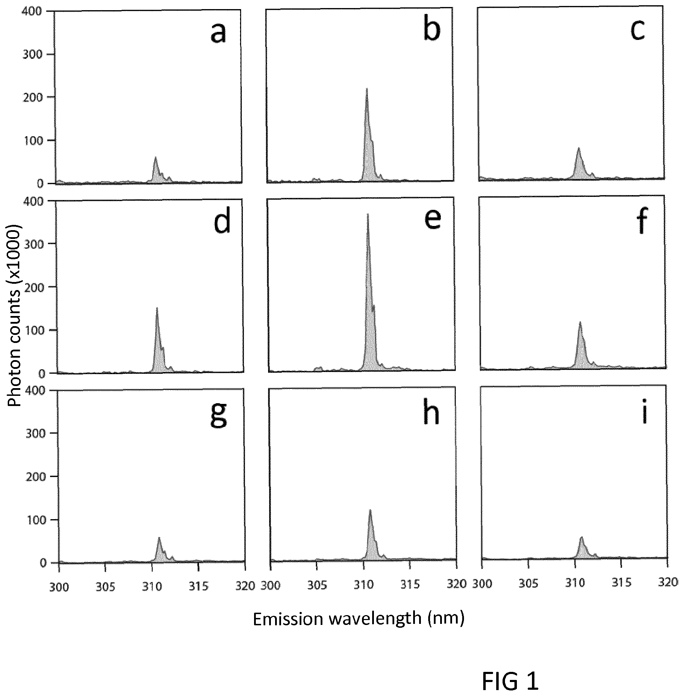

FIGURE 1 shows upconversion emission spectra of NaYF₄ doped with Gd³⁺ and Ho³⁺ upon excitation at 448 nm.

### DETAILED DESCRIPTION OF THE INVENTION

### Material and phosphor

The invention provides a material capable of emitting radiation in the UV-B wavelength range (between 290 and 320 nm), preferably in the wavelength range between 305 nm and 315 nm, upon excitation by radiation in the wavelength range between 400 nm and 495 nm.

More preferably, the material is capable of emitting radiation in the UV-B wavelength range (between 290 and 320 nm), preferably in the wavelength range between 305 nm and 315 nm, upon excitation by radiation in the wavelength range between 440 nm and 470 nm.

The material according to the invention may be a phosphor or may comprise a phosphor.

The phosphor is capable of emitting radiation in the UV-B wavelength range (between 290 and 320 nm), preferably in the wavelength range between 305 nm and 315 nm.

Preferably, the phosphor is capable of emitting radiation in the UV-B wavelength range (between 290 and 320 nm), preferably in the wavelength range between 305 nm and 315 nm, upon excitation by radiation in the wavelength range between 400 nm and 495 nm.

More preferably, the phosphor is capable of emitting radiation in the UV-B wavelength range (between 290 and 320 nm), preferably in the wavelength range between 305 nm and 315 nm, upon excitation by radiation in the wavelength range between 440 nm and 470 nm.

As disclosed hereinafter, a phosphor capable of emitting radiation in the UV-B wavelength range (between 290 and 320 nm), preferably in the wavelength range between 305 nm and 315 nm, upon excitation by radiation in other wavelength ranges may also be used, for instance when such phosphor is comprised in a material further comprising a sensitizer.

Preferably the material and/or phosphor exhibits an emission peak in the UV-B wavelength range (between 290 and 320 nm), preferably in the wavelength range between 305 nm and 315 nm, upon excitation by radiation. Preferably, the material and/or phosphor exhibits such emission peak upon excitation by radiation in the wavelength range between 400 nm and 495 nm, more preferably in the wavelength range between 440 nm and 470 nm. Preferably, the material and/or phosphor exhibits an absorption peak in the wavelength range between 400 nm and 495 nm, more preferably in the wavelength range between 440 nm and 470 nm.

As used throughout the entire application, radiation in the UV-B wavelength range refers to radiation in the wavelength range between 290 and 320 nm. Preferably, the radiation in the UV-B wavelength range, or in the preferred wavelength ranges between 305 nm and 315 nm, or between 310 nm and 315 nm, is narrow band radiation in these wavelength ranges. Narrow band radiation refers to radiation having a peak (in the respective wavelength range) of which the full-width half maximum is less than 10 nm.

The preferred wavelength range (for radiation emitting from the material and/or phosphor) between 305 nm to 315 nm as disclosed herein is particularly preferred in view of advantages offered in the treatment of skin, in particular psoriasis. The preferred wavelength range between 440 nm and 470 nm (for excitation of the material and/or phosphor), is particularly preferred as it offers even further safety for the skin. Preferably these ranges are applied in combination.

The skilled person will understand that phosphors disclosed herein are based on the principle of upconversion. Thus, the phosphor disclosed herein may also be referred to as an upconversion phosphor. Upconversion involves tThe creation of a high energy photon (e.g. a UV-B photon) by the absorption of multiple lower energy photons (e.g. blue photons). Whilst the phenomenon of upconversion, has been known, for instance the upconversion from NIR to the visible range, the present invention shows that upconversion from the 400 nm to 495 nm wavelength range to the UV-B wavelength range is possible.

Preferably, the phosphor comprises a host lattice doped with lanthanide ions. The lanthanides involve the group of elements from La³⁺ (4f⁰) to Lu³⁺ (4f¹⁴) where the inner 4f shell is (partly) filled with n electrons after the outer 5s and 5p shells are filled. Efficient shielding of the 4fⁿ shell by the outer 5s² and 5p⁶ shells results in very sharp absorption and emission transition lines and weak coupling with lattice vibrations for 4fⁿ-4fⁿ transition. The latter leads to limited quenching by lattice vibrations and very high luminescence quantum efficiencies, up to almost 100%. Since the electron configuration does not change for an intraconfigurational 4fⁿ-4fⁿ transition these transitions are parity forbidden, resulting in weak absorption strengths and long luminescence life times for 4fⁿ-4fⁿ transitions. The high efficiency and the rich energy level structure make that lanthanides are particularly suitable for spectral conversion. For upconversion, the long life time of excited states is advantageous as it allows for energy transfer (ET) to lanthanides ions in an excited state to an even higher excited state.

Preferably, the host lattice is doped with Gd³⁺ ions. For upconversion of radiation in the 400 nm to 495 nm wavelength range (e.g. blue light) to UV-B emission in the 305-315 nm range Gd³⁺ is ideal as an emitter. Gd³⁺ has the 4f⁷ configuration. The half-filled shell configuration is particularly stable and the first excited state (⁶P_{7/2}) is about 32000 cm⁻¹ above the ⁸S_{7/2} ground state. This energy difference corresponds to ∼312 nm.

Preferably, the phosphor has an efficient population of the Gd^{3+ 6}P_{7/2} excited state under excitation with radiation in the 400 nm to 495 nm wavelength range (e.g. blue light). Preferably, ions leading to upconversion resulting in excitation of the ⁶P_{7/2} level of Gd³⁺, satisfy the following criteria
- The ion preferably has an emitting level between 32 000 and 40 000 cm⁻¹ to allow for ET to the ⁶P_{J}, ⁶I_{J} or ⁶D_{J} states of Gd³⁺.
- The ion has a long lived excited state in the ∼16 000 to 22 000 cm⁻¹ energy range to allow for upconversion to the high energy state that can transfer the energy to Gd³⁺.
Preferred are combinations Gd³⁺ with Ho³⁺ (4f¹⁰), Tm³⁺ (4f¹²), Er³⁺ (4f¹¹) and/or Dy³⁺ (4f⁸)

Preferably, the phosphor comprises a host lattice doped with (i) first lanthanide ions which are Gd³⁺ ions and (ii) second lanthanide ions selected from the group consisting of Ho³⁺, Tm³⁺, Er³⁺ and Dy³⁺ and combinations thereof. Preferably, said second lanthanide ions are Ho³⁺ ions.

The first lanthanide ions may be present at any suitable doping concentration. Preferably, the first lanthanide ions are present at a doping concentration of at least 10 mol.%, preferably at least 16 mol.%, more preferably at least 20 mol.%. This is found to result in an increase of the intensity of the emission peak. There is no particular upper limit for the doping concentration of the first lanthanide ions. Of course the skilled person understands that the sum concentration of dopants and unsubstituted rare earth ions of the host lattice will be 100 %.

The second lanthanide ions may be present at any suitable doping concentration. Preferably, the second are present at a doping concentration of at least 0.5 mol.%, more preferably at least 1.5 mol.%, more preferably between 1.5 and 10 mol.%, and even more preferably between 2 and 6 mol.%. Increasing the doping concentration to above the preferred lower limits was found to result in an increase of the intensity of the UV-B emission peak. However, a doping concentration of the second lanthanide ions above the preferred upper limits was found to result in a decrease of the intensity of the emission peak.

Any suitable host lattice may be used. Preferably, the host lattice is an oxide, fluoride, nitride, chloride, sulfide, selenide, oxyfluoride, oxychloride, oxynitride oxysulfide, oxyselenide, fluorochloride, fluorobromide or another inorganic host material in which the optically active lanthanide ions can be incorporated. Preferably the host lattice has no groups with high energy vibrations (phonons) exceeding energies of ∼750 cm⁻¹. High energy vibrations can induce vibrational relaxation processes, known as multi-phonon relaxation, that can reduce the efficiency. There is a large variety of suitable host lattices known, for example yttriumoxide (Y₂O₃), lanthanumoxide (La₂O₃), lutetium oxide (Lu₂O₃), sodiumyttriumoxide (NaYO₂), sodiumlutetiumoxide (NaLuO₂), lanthanumfluoride (LaF₃), yttriumfluoride (YF₃), lutetiumfluoride (LuF₃), sodiumyttriumfluoride (NaYF₄), sodiumlutetiumfluoride (NaLuF₄), lihiumyttriumfluoride (LiYF₄), yttriumoxyfluoride (YOF), yttriumoxychloride (YOCl), yttium aluminate (YAlO₃), yttriumaluminumgarnet (Y₃Al₅O₁₂), yttriumoxysulfide (Y₂O₂S), strontiumyttriumsiliconnitride (SrYSi₄N₇), yttriumsiliconoxynitride (Y₄Si₂O₇N₂). Y₂O₃, NaYF₄ NaLuF₄, Lu₂O₃, Y₂O₂S en YF₃, Lu₂O₂S, or LuF₃ may for instance be used. NaYF₄ is a preferred host lattice.

Preferably, the host lattice is not doped with Yb³⁺.

Based on the principles disclosed herein, the skilled person is able to obtain further upconversion phosphors having the spectral characteristics according to the invention.

The phosphor may be in any suitable form. Preferably the phosphor according to the invention is in the form of microcrystalline particles or nanocrystalline particles.

The phosphor may be prepared in any suitable manner and using techniques for preparing phosphors known to the skilled person. For instance using solid state synthesis methods to prepare microcrystalline phosphors. Precursor materials may be thoroughly mixed by grinding in a planetary ball mill or using a pestle and mortar. The mixed precursors may be transferred to a reaction crucible (for example an aluminum oxide container) and heated in a furnace to the reaction temperature which may vary, depending on the phosphor host material, between for instance ∼500 °C and ∼1800 °C. Often the heating is performed in a controlled atmosphere (for example nitrogen gas). Alternative synthesis methods for microcrystalline phosphors involve wet-chemical synthesis routes such as co-precipitation. In this method, precursors are first dissolved, for example in water. Mixing the solutions of precursors results in the formation of the insoluble phosphor material which precipitates. After washing and drying and possibly a heat treatment of the precipitate in a furnace the phosphor is obtained.

Synthesis methods for nanocrystalline phosphors may include combustion synthesis and colloidal synthesis techniques. In combustion synthesis precursors are mixed with a fuel (for example glycine). Heating the mixture in air in a furnace initiates a rapid self-propagating combustion reaction of the fuel and precursors giving rise to high reaction temperatures in which the precursors react to nanocrystalline phosphor particles. In colloidal synthesis techniques controlled growth of nanoparticles can be realized by heating precursors in a solvent in the presence of coordinating ligands. Heating induces the formation of initial seed crystals which grow to larger nanometer sized particles. Particle growth and size are controlled by passivating surface ligands, temperature and precursor concentrations.

In a further aspect, the invention provides a phosphor comprising a host lattice doped with at least (i) first lanthanide ions which are Gd³⁺ ions and (ii) second lanthanide ions selected from the group consisting of Ho³⁺, Tm³⁺, Er³⁺, Dy³⁺ and combinations thereof. Preferably said second lanthanide ions are Ho³⁺ ions. All further preferred features and characteristics as described above, e.g. with respect to the dopants, doping concentrations, host lattice, form and preparation process, apply to the phosphor according to this aspect *mutatis mutandis.*

### Sensitizer

The material according to the invention may comprise (I) a phosphor; and (II) a sensitizer.

Preferred features and characteristics of the phosphor have been described hereinabove.

The use of a sensitizer may increase the upconversion efficiency and intensity of radiation emitted by the phosphor may be increased by applying a sensitizer. Also, it may facilitate the use of a less intense and/or broad band excitation source. In the event of treatment of the skin, the presence of a sensitizer provides the further advantage that it may serve as a further control of the dosage of UV-B radiation received by the patient.

Any suitable sensitizer may be used. Suitable sensitizers include sensitizers of which the emission spectrum at least partly overlaps with the excitation spectrum of the phosphor.

Preferably, said sensitizer is capable of absorbing radiation in the wavelength range between 400 nm and 495 nm, more preferably in the wavelength range between 440 nm and 470 nm.

Preferably, the sensitizer comprises molecules, preferably organic molecules or metal-organic molecules, wherein at least part said molecules are covalently bound to the phosphor. Preferably, the phosphor is in the form of nanoparticles, the sensitizer comprises molecules, preferably organic molecules, wherein at least part of the molecules is covalently bound to the nanoparticles.

Preferably, the sensitizer is a dye, more preferably a fluorescent dye. Dyes are well known to the skilled person, and the skilled person will able to find dyes of which the emission spectrum at least partly overlaps with the excitation spectrum of the phosphor. Preferably, the dye has a high absorption strength (ε > 10³ M⁻¹.cm⁻¹) in the wavelength range between 400 nm and 495 nm, more preferably in the wavelength range between 440 nm and 470 nm.

Preferred dyes contain a conjugated chain of methine units or aromatic compounds (π-systems), including poly(fluorene), poly(*para*-phenylene), poly(*p-*phenyleneethynylene), poly(p-phenylenevinylene), poly(thiophene), porphyrine, phenoxazine, naphtacene and their derivatives. Coumarins, perylenes, thiophenes, porphyrines, pyridines, polyfluorenes, phenylenes are preferred. These dyes show strong absorption in the 400-495 nm spectral region and an efficient emission.

Preferably, the dye contains functional groups. The functional group may be selected to effect binding of the dye to the phosphor, in particular to the phosphor in the form of nanoparticles. Preferred functional groups include succinimidyl esters, hydrazide, maleimide, azide and carboxylic acid groups. Carboxylic acid groups are preferred.

The phosphor, in particular the phosphor in the form of nanoparticles may be coated with carboxylic acid-functionalized dye molecules by a simple partial exchange reaction with surface-bound oleylamine. The process described in Zou et al, Broadband dye-sensitized upconversion of near-infrared, Nature Photonics, vol. 6, August 2012, p. 560-564 may advantageously be used.

### Topical compositions

The invention also provides a composition comprising the material and/or phosphor according to the invention. Preferably, the composition is a topical composition. The topical composition may further comprise any suitable topically acceptable agent or material. Suitable topically acceptable agents include for instance fatty acids, fatty esters, waxes, oils, vegetable oils (e.g. corn oil, canola oil and soybean oil), mineral oils, triglycerides, long chain alcohols, glycerol, silicones, emulsions (e.g. water and oil, oil and wax, wax and water), antiseptics, astringents, and combinations thereof. Suitable materials include for instance silica sol-gels, polystyrenes, silicon-based polymers, polyacrylates, polyurethanes, polycarbonates, and combinations thereof. Preferably, the absorption spectrum of the topically acceptable agent or material exhibit no or substantially no absorption in the wavelength range between 305 nm and 315 nm. Emollients (e.g. Vaseline oil), and mineral oils, glycerol are preferred, in particular for the treatment of psoriasis. The composition may include further functional ingredients to add other therapeutic effects. Such ingredients could be e.g. Cell growth inhibitors (e.g. Dithranol), Vitamin D derivatives (e.g. Calcipotriol, Tacalcitol, Calcitriol), topical corticosteroids or coal tar derivates.

Preferably, the topical composition is a cream, spray, foam, gel (optionally comprised in a gel pad), lotion or ointment.The material, phosphor, and/or topical composition , may be included in a sticking fabric, patch or plaster.A topical composition may be applied locally. This facilitates applying the UV-B radiation locally and in a controlled way.

### Systems, methods, kit of parts, uses

The invention further provides a system for generating UV-B radiation, said system comprising:
(a) the material and/or phosphor according to the invention or a composition comprising the material and/or phosphor according to the invention; and
(b) a light source capable of emitting radiation in the wavelength range between 400 nm and 495 nm, preferably in the wavelength range between 440 and 470 nm.

The invention further provides a kit of parts comprising:
a) the material and/or phosphor according to the invention or a composition comprising the material and/or phosphor according to the invention; and
(b) a light source capable of emitting radiation in the wavelength range between 400 nm and 495 nm, preferably between 440 nm and 470 nm.

The invention further provides a method of generating UV-B radiation, said method comprising:
(a) providing the material and/or phosphor according to the invention or a composition comprising the material and/or phosphor according to the invention; and
(b) exposing said material and/or phosphor or a composition comprising the material and/or phosphor to radiation in the wavelength range between 400 nm and 495 nm, preferably in the wavelength range between 440 nm and 470 nm.
Said exposing may be performed in any suitable manner, in particular under conditions such that the material and/or phosphor is excited and said UV-B radiation is emitted.

The invention further provides a method of exposing cells and/or cell tissue, preferably skin, to UV-B radiation, said method comprising generating UV-B radiation according to the method for generating UV-B radiation according to the invention, and exposing said cells and/or cell tissue to said UV-B radiation. Preferably said method comprises applying said the material and/or phosphor according to the invention or said composition comprising the material and/or phosphor according to the invention to the cells or cell tissue; and exposing the applied material or composition to said radiation in the wavelength range between 400 nm and 495 nm, preferably in the wavelength range between 440 nm and 470 nm.

Said method may for instance be at least one of cosmetic treatment, prophylactic treatment, therapeutic treatment; phototherapy and/or photodynamic therapy.

Said method may for instance be a method for therapeutic treatment, preferably for the treatment of psoriasis, vitiligo, atopic dermatitis, acne or pruritus, more preferably for the treatment of psoriasis.

In an embodiment the method or use is not treatment of the human or animal body by surgery or therapy and/or is not practiced on the human or animal body.

The invention further provides the use of the material and/or phosphor according to the invention or a composition comprising the material and/or phosphor according to the invention for the generation of radiation in the UV-B wavelength range (between 290 and 320 nm), preferably in the wavelength range between 305 nm and 315 nm, upon excitation by radiation in the wavelength range between 400 nm and 495 nm, preferably in the wavelength range between 440 and 470 nm.

The invention further provides the material and/or phosphor according to the invention or a composition comprising the material and/or phosphor according to the invention for use in a therapeutic treatment, preferably for the treatment of psoriasis, vitiligo, atopic dermatitis, acne or pruritus, more preferably for the treatment of psoriasis, wherein cells and/or cell tissue, preferably skin, are exposed to UV-B radiation generated by exciting the phosphor with radiation in the wavelength range between 400 nm and 495 nm, preferably between 440 and 470 nm.

### Light sources

Any suitable light source capable of emitting radiation in the wavelength range between 400 nm and 495 nm, preferably in the wavelength range between 440 nm and 470 nm, may be used. The light source may be adapted to emit radiation capable of exciting the material and/or phosphor by based on principles known in the art. Suitable light sources include light sources having an emission spectrum in the wavelength range between 400 nm and 495, preferably between 440 nm and 470 nm, which emission spectrum at least partly overlaps with the excitation spectrum of the material and/or phosphor in the wavelength range between 400 nm and 495, preferably between 440 nm and 470 nm.

Any suitable artificial light source may be used. The light source may be a light emitting diode (LED) or laser, e.g. a diode laser or pulsed laser. Broadly available, highly efficient and cheap light sources are for example InGaN based blue LEDs.

In the event of treatment of the skin, the light source may be placed in direct proximity to the treatment area of the skin, or in a remote location to the skin. The light source may have a flexible or partly flexible body, to adjust its radiating surface to the treatment surface. The light source may have optical means to focus the light to the treatment area of the skin, e.g. reflectors, optical lenses or light shielding elements. The light source may have optical means to even the light output over the radiating surface, e.g. by light diffusing optical elements and/or diffusing materials. The light source may have means to attach it temporarily at the patient's body, e.g. the limbs, in direct proximity of the treatment area. The light source may have means for monitoring the blue light dose applied to the treatment area, e.g. by a timer and flux density adjustment. The light source may have means for monitoring the UV light dose applied to the treatment area, e.g. by UV-light sensors. The light source may have means for sensing the intensity of the disease, e.g. thickness and/or colour of psoriasis plaques, e.g. by optical sensors. These means may be used to observe the course of the therapy, or to calculate the dose to be applied. The light source may have means to adjust the distance and orientation of the light source to a specific area of the treatment surface.

A person skilled in the art of narrow-band UV-B (peak wavelength between 305-315 nm) phototherapy will be able to determine an effective dose to treat a specific skin disease, e.g. based on the patient's skin type. For example, for a targeted phototherapy of psoriasis lesions, a usual starting dose of moderate to mild pronounced lesions for skin type 1-3 is 500 mJ/cm² of narrow-band UVB. This dose will be subsequently increased after the first treatment, typically according to the erythema reaction of the skin or according to improvements to the lesions. For darker skin tones, higher starting doses are recommended. The preferred maximum dose applied to psoriasis lesions, which is typically reached within 10 treatments, is 5000 mJ/cm² for skin type 6 (black skin). For psoriasis lesions with not clearly define borders, thus where partly non-infected areas of healthy skin are included within the lesions, it may be beneficial to start with lower doses of narrow band UVB like suggested for common narrow-band UVB phototherapy. The lowest recommended starting dose for such non-targeted, common treatment is 130 mJ/cm² of narrow band UVB. According to the above described, recommended range of 130 mJ/cm² - 5000 mJ/cm² narrow band UVB radiation applied to the skin, the light source providing the blue light for exciting the upconverter phosphor should provide an sufficient amount of blue light to enable the described doses of narrow band UVB. For example, if the total external quantum efficiency of the topical composition in combination with the light source is 1% (defined as the fraction of UVB light reaching the skin compared to the amount of blue light reaching the upconverter material), a light source must be able to supply min. 13 J/cm², and max. 500 J/cm² of blue light to cover the full range of doses. If the external quantum efficiency of the topical composition is higher or lower than the above exemplarily used 1%, then the required blue light dose may be accordingly lower or higher, to achieve the same resulting dose of narrow band UVB light. To achieve such doses of blue light, a person skilled in the art will calculate the applied blue light dose by taking into account the flux density (optical watt per cm²) of blue light supplied by the device, and the time how long the device is switched on. For example, if the required blue light dose is 50 J/cm², and the light device is able to deliver in average a flux density of 200 mW/cm² of blue light, the device may be switched on for 250 seconds to supply the required blue light dose.

It has been observed that a flux density of 50-280 mW/cm² is a beneficial range of flux density for a blue light device, most beneficial between 80-200 mW/cm². This range was chosen since a lower flux density will unnecessarily increase the treatment time, and a higher flux density potentially could induce unpleasant heat in the skin. It has been observed that it is beneficial to pulse the blue light source, to decrease the heat induction in the skin. For example, when the blue light is pulsed with 50 Hz frequency, resulting in 10 ms long pulses of 100% light intensity followed by 10 ms off-time (0% light), that the maximum blue light dose accepted by the patient regarding the perceived heat was up to about 60% higher compared to constant light (acceptable limits where 150 mW/cm² constant light compared to 240 mW/cm² average flux density of pulsed light). This means, pulsed light potentially can decrease the treatment time since the flux density of the blue light source can be increased due to lower heat induction in the skin. This is especially beneficial if it cannot be prevented that the blue light also irradiates on non-effected, healthy skin which is not covered by the topical composition.

The invention will now further be illustrated using the following examples, without however being limited thereto.

### EXAMPLES

### Example 1: synthesis

The synthesis method of Linda Aarts et al.[1] was used as a starting point for the synthesis of phase- pure *β*-NaYF₄. This synthesis consists of mixing the NaF and YF₃ precursors with the fluoride precursors of the lanthanide dopants and with NH4F. Precursors are slightly hygroscopic and were therefore dried at ∼120 °C prior to the synthesis to avoid a non-stoichiometric mixture. This mixture is sintered in a tube oven under nitrogen atmosphere, first at 300 °C for two hours and then at 550 °C for three hours. At the entrance of the tube oven a crucible with NH4F is placed, to create a fluoride-rich atmosphere.

When using this synthesis method for NaYF₄:Gd³⁺,Ho³⁺ with a high Gd³⁺ dopant concentration, significant amounts of unreacted GdF₃ precursor remain. Better results were obtained when the mixture was sintered for ten hours at 600 °C and when a 20% excess of NaF was used.

Nine samples with various concentrations of Gd³⁺ and Ho³⁺ were synthesized: samples with 1% Ho³⁺ and 10, 25 or 99% Gd³⁺; samples with 3% Ho³⁺ and 10, 25 or 97% Gd³⁺; samples with 7% Ho³⁺ and 10, 25 or 93% Gd³⁺. X-ray diffraction was applied to investigate the phase purity of the material. Good agreement between the measured diffractograms and the reference diffractograms was found, which means that the synthesis of phase-pure β-NaYF₄ has succeeded.

### Example 2: generation of UV-B radiation

For all nine samples it was investigated whether excitation with blue light results in UV emission. High-intensity excitation was done with Opotek Opolette 355 laser system giving ∼1 mJ pulses at 448 nm with a pulse duration of 10 ns and a repetition rate of 10 Hz. The output of the laser was directed using a prism towards a microcrystalline sample of the upconversion material (NaYF₄ doped with Ho³⁺ and Gd³⁺). The upconversion phosphor was pressed in a sample holder placed in an Edinburgh Instruments FLS 920 Spectrofluorometer with a Hamamatsu R928 photomultiplier tube. Upconversion emission spectra were measured by scanning the emission monochromator of the FLS 920 Spectrofluorometer in the UV spectral region while exciting the phosphor with the OPO laser system at 448 nm in the blue spectral region. All spectra were recorded under identical conditions so that the relative intensities of the UV emission peak reflect the efficiency of the upconversion efficiency in the phosphors.

Blue-to-UV upconversion was found in all nine samples. Figure 1 shows the UV emission spectra of all samples upon excitation with blue 448 nm light in the ⁵F₁/⁵G₆ level of Ho³⁺, wherein
a. 1% Ho³⁺, 0% Gd³⁺
b. 1% Ho³⁺, 25% Gd³⁺
c. 1% Ho³⁺, 99% Gd³⁺
d. 3% Ho³⁺, 10% Gd³⁺
e. 3% Ho³⁺, 25% Gd³⁺
f. 3% Ho³⁺, 97% Gd³⁺
g. 7% Ho³⁺, 10% Gd³⁺
h. 7% Ho³⁺, 25% Gd³⁺
i. 7% Ho³⁺, 93% Gd³⁺

The UV emission intensity depends on the concentration of the Gd³⁺ and Ho³⁺ dopants. Of all the synthesized samples, the material with 3% Ho³⁺ and 25% Gd³⁺ yields the highest UV emission intensity.

### References

[1] L. Aarts, B. M. van der Ende, and A. Meijerink, Journal of Applied Physics 106, 023522 (2009).

## Claims

1. A material capable of emitting radiation in the UV-B wavelength range (between 290 and 320 nm), preferably in the wavelength range between 305 nm and 315 nm, upon excitation by radiation in the wavelength range between 400 nm and 495 nm.

2. The material according to claim 1, wherein said material is or comprises a phosphor, preferably wherein said phosphor comprises a host lattice doped with lanthanide ions.

3. The material according to claim 2, wherein said host lattice is doped with at least (i) first lanthanide ions which are Gd³⁺ ions and (ii) second lanthanide ions selected from the group consisting of Ho³⁺, Tm³⁺, Er³⁺ and Dy³⁺ and combinations thereof.

4. The material according to claim 3, wherein said second lanthanide ions are Ho³⁺ ions.

5. The material according to claim 3 or 4, wherein said first lanthanide ions are present at a doping concentration of at least 10 mol.%, preferably at least 16 mol.%, more preferably at least 20 mol.%.

6. The material according to any one of clams 3 to 5, wherein said second lanthanide ions are present at a doping concentration of at least 1.5 mol.%, preferably between 1.5 and 10 mol.%, more preferably between 2 and 6 mol.%.

7. The material according to any one of claims 2 to 6, wherein said phosphor comprises a host lattice which is selected from the group consisting of oxides, fluorides, nitrides, chlorides, sulfides, selenides, oxyfluorides, oxychlorides, oxynitrides, oxysulfides, oxyselenides, fluorochlorides, and fluorobromides or another inorganic host material in which the optically active lanthanide ions can be incorporated.

8. The material according to claim 7, wherein the host lattice is NaYF₄.

9. The material according to any preceding claim, said material comprising (I) a phosphor; and (II) a sensitizer.

10. The material according to claim 9, wherein said phosphor is capable of emitting radiation in the UV-B wavelength range (between 290 and 320 nm), preferably in the wavelength range between 305 nm and 315 nm, said sensitizer is capable of absorbing radiation in the wavelength range between 400 to 495 nm; and wherein the emission spectrum of the sensitizer at least partly overlaps with the excitation spectrum of the phosphor.

11. The material according to claim 9 or 10, wherein said sensitizer is a dye.

12. The material according to any one of claims 9 to 11, wherein the phosphor is in the form of nanoparticles, the sensitizer comprises molecules, preferably organic molecules, wherein at least part of the molecules is covalently bound to the nanoparticles.

13. A topical composition, preferably a cream, spray, foam, gel, lotion or ointment, said topical composition comprising the material according to any preceding claim.

14. A system for generating UV-B radiation, said system comprising
(a) a material according to any one of claims 1 to 12 or a composition comprising the material according to any one of claims 1 to 12; and
(b) a light source capable of emitting radiation in the wavelength range between 400 nm and 495 nm.

15. A method of generating UV-B radiation, said method comprising
(a) providing a material according to any one of claims 1 to 12 or a composition comprising the material according to any one of claims 1 to 12; and
(b) exposing said material or composition to radiation in the wavelength range between 400 nm and 495 nm.

16. A kit of parts comprising
a) a material according to any one of claims 1 to 12 or a composition comprising the material according to any one of claims 1 to 12; and
(b) a light source capable of emitting radiation in the wavelength range between 400 nm and 495 nm.

17. Use of a material according to any one of claims 1 to 12 or a composition comprising the material according to any one of claims 1 to 12 for the generation of radiation in the UV-B wavelength range (between 290 and 320 nm), preferably in the wavelength range between 305 nm and 315 nm, upon excitation by radiation in the wavelength range between 400 nm and 495 nm.

18. A system according to claim 14, a method according to claim 15, or a kit of parts according to claim 16, wherein said composition is a topical composition, preferably a cream, spray, pad foam, gel, lotion or ointment.

19. A method of exposing cells and/or cell tissue, preferably skin, to UV-B radiation, said method comprising generating UV-B radiation according to the method according to claim 15 or 18; and exposing said cells and/or cell tissue to said UV-B radiation.

20. The method according to claim 19, wherein said method comprises applying said material or said composition to the cells or cell tissue; and exposing the applied material or composition to said radiation in the wavelength range between 400 nm and 495 nm.

21. The method according to claim 19 or 20, wherein said method is at least one of cosmetic treatment, prophylactic treatment, therapeutic treatment; phototherapy and/or photodynamic therapy.

22. The method according to any one of claims 19 to 21, wherein said method is a method for therapeutic treatment, preferably for the treatment of psoriasis, vitiligo, atopic dermatitis, acne or pruritus, more preferably for the treatment of psoriasis.

23. The method or use according to any one of claims 15 to 22, wherein the method or use is not treatment of the human or animal body by surgery or therapy and/or is not practiced on the human or animal body.

24. The material according to any one of claims 1 to 12 or a composition comprising the material according to any one of claims 1 to 12 for use in a therapeutic treatment, preferably for the treatment of psoriasis, vitiligo, atopic dermatitis, acne or pruritus, more preferably for the treatment of psoriasis, wherein cells and/or cell tissue, preferably skin, are exposed to UV-B radiation generated by exciting the phosphor with radiation in the wavelength range between 400 nm and 495 nm.

25. A phosphor comprising a host lattice doped with at least (i) first lanthanide ions which are Gd³⁺ ions and (ii) second lanthanide ions selected from the group consisting of Ho³⁺, Tm³⁺, Er³⁺, Dy³⁺ and combinations thereof.

26. The phosphor according to claim 25, wherein said second lanthanide ions are Ho³⁺ ions.

27. The phosphor according to claim 25 or 26, wherein said first lanthanide ions are present at a doping concentration of at least 10 mol.%, preferably at least 16 mol.%, more preferably at least 20 mol.%.

28. The phosphor according to any one of clams 24 to 27, wherein said second lanthanide ions are present at a doping concentration of at least 1.5 mol.%, preferably between 1.5 and 10 mol.%, more preferably between 2 and 6 mol.%.

29. The phosphor according to any one of claims 24 to 28, wherein said phosphor comprises a host lattice which is selected from the group consisting of oxides, fluorides, nitrides, chlorides, sulfides, selenides, oxyfluorides, oxychlorides, oxynitrides, oxysulfides, oxyselenides, fluorochlorides, and fluorobromides.

30. The phosphor according to claim 29, wherein the host lattice is NaYF₄.

31. A material, said material comprising (I) a phosphor according to any one of claims 24 to 30; and (II) a sensitizer.

32. The material according to claim 31, wherein said phosphor is capable of emitting radiation in the UV-B wavelength range (between 290 and 320 nm), preferably in the wavelength range between 305 nm and 315 nm, said sensitizer is capable of absorbing radiation in the wavelength range between 400 to 495 nm; and wherein the emission spectrum of the sensitizer at least partly overlaps with the excitation spectrum of the phosphor.

33. The material according to claim 31 or 32, wherein said sensitizer is a dye.

34. The material according to any one of claims 31 to 33, wherein the phosphor is in the form of nanoparticles, the sensitizer comprises molecules, preferably organic molecules, wherein at least part of the molecules is covalently bound to the nanoparticles.

35. A topical composition, preferably a cream, spray, foam, gel, lotion or ointment, said topical composition comprising the phosphor or material according to any preceding claim.

36. A system for generating UV-B radiation, said system comprising:
(a) a phosphor according to any one of claims 25 to 30, a composition comprising the phosphor according to any one of claims 25 to 30, a material according to any one of claims 31 to 35, or a composition comprising the material according to any one of claims 31 to 35; and
(b) a light source capable of emitting radiation in the wavelength range between 400 nm and 495 nm.

37. A method of generating UV-B radiation, said method comprising
(a) providing a phosphor according to any one of claims 25 to 30, a composition comprising the phosphor according to any one of claims 25 to 30, a material according to any one of claims 31 to 35, or a composition comprising the material according to any one of claims 31 to 35; and
(b) exposing said phosphor, material or composition to radiation in the wavelength range between 400 nm and 495 nm.

38. A kit of parts comprising
a) a phosphor according to any one of claims 25 to 30, a composition comprising the phosphor according to any one of claims 25 to 30, a material according to any one of claims 31 to 35, or a composition comprising the material according to any one of claims 31 to 35; and
(b) a light source capable of emitting radiation in the wavelength range between 400 nm and 495 nm.

39. Use of a phosphor according to any one of claims 25 to 30, a composition comprising the phosphor according to any one of claims 25 to 30, a material according to any one of claims 31 to 35, or a composition comprising the material according to any one of claims 31 to 35 for the generation of radiation in the UV-B wavelength range (between 290 and 320 nm), preferably in the wavelength range between 305 nm and 315 nm, upon excitation by radiation in the wavelength range between 400 nm and 495 nm.

40. A system according to claim 36, a method according to claim 37, or a kit of parts according to claim 38, wherein said composition is a topical composition, preferably a cream, spray, foam, gel, lotion or ointment.

41. A method of exposing cells and/or cell tissue, preferably skin, to UV-B radiation, said method comprising generating UV-B radiation according to the method according to claim 37 or 40; and exposing said cells and/or cell tissue to said UV-B radiation.

42. The method according to claim 41, wherein said method comprises applying said material or said composition to the cells or cell tissue; and exposing the applied material or composition to said radiation in the wavelength range between 400 nm and 495 nm.

43. The method according to claim 41 or 42, wherein said method is at least one of cosmetic treatment, prophylactic treatment, therapeutic treatment; phototherapy and/or photodynamic therapy.

44. The method according to any one of claims 41 to 43, wherein said method is a method for therapeutic treatment, preferably for the treatment of psoriasis, vitiligo, atopic dermatitis, acne or pruritus, more preferably for the treatment of psoriasis.

45. The method or use according to any one of claims 37 to 44, wherein the method or use is not treatment of the human or animal body by surgery or therapy and/or is not practiced on the human or animal body.

46. The phosphor according to any one of claims 25 to 30, a composition comprising the phosphor according to any one of claims 25 to 30, a material according to any one of claims 31 to 35, or a composition comprising the material according to any one of claims 31 to 35 for use in a therapeutic treatment, preferably for the treatment of psoriasis, vitiligo, atopic dermatitis, acne or pruritus, more preferably for the treatment of psoriasis, wherein cells and/or cell tissue, preferably skin, are exposed to UV-B radiation generated by exciting the phosphor with radiation in the wavelength range between 400 nm and 495 nm.
